(19) **Europäisches Patentamt / European Patent Office / Office européen des brevets**

(11) **EP 4 066 829 A1**

(12) **EUROPEAN PATENT APPLICATION**
published in accordance with Art. 153(4) EPC

(43) Date of publication:
**05.10.2022   Bulletin 2022/40**

(21) Application number: **20894038.7**

(22) Date of filing: **20.11.2020**

(51) International Patent Classification (IPC):
**A61K 31/4045** (2006.01)   **A61K 9/70** (2006.01)
**A61K 47/02** (2006.01)   **A61K 47/32** (2006.01)
**A61K 47/34** (2017.01)   **A61P 25/00** (2006.01)
**A61P 25/16** (2006.01)

(52) Cooperative Patent Classification (CPC):
**A61K 9/70; A61K 31/4045; A61K 47/02;**
**A61K 47/32; A61K 47/34;** A61P 25/00; A61P 25/16

(86) International application number:
**PCT/JP2020/043382**

(87) International publication number:
**WO 2021/106782 (03.06.2021 Gazette 2021/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.11.2019   JP 2019213165**

(71) Applicant: **Hisamitsu Pharmaceutical Co., Inc.**
**Tosu-shi, Saga 841-0017 (JP)**

(72) Inventors:
• **UCHIDA Naoyuki**
  **Tsukuba-shi, Ibaraki 305-0856 (JP)**
• **AMANO Satoshi**
  **Tsukuba-shi, Ibaraki 305-0856 (JP)**

(74) Representative: **Grünecker Patent- und Rechtsanwälte**
**PartG mbB**
**Leopoldstraße 4**
**80802 München (DE)**

(54) **METHOD FOR IMPROVING HOLDING POWER OF ADHESIVE AGENT LAYER IN ROPINIROLE-CONTAINING TRANSDERMAL PATCH, AND ROPINIROLE-CONTAINING TRANSDERMAL PATCH HAVING IMPROVED HOLDING POWER**

(57)    A method for improving holding power of an adhesive agent layer in a ropinirole-containing patch comprising a backing layer and the adhesive agent layer, wherein the adhesive agent layer contains at least one selected from the group consisting of ropinirole and a pharmaceutically acceptable salt thereof and an adhesive agent, the method comprising:
causing the adhesive agent layer to further contain a sodium salt of carbonic acid.

**Description**

[Technical Field]

The present invention relates to a method for improving holding power of an adhesive agent layer in a ropinirole-containing patch and a ropinirole-containing patch with improved holding power, and more specifically to a method for improving holding power of an adhesive agent layer in a ropinirole-containing patch containing ropinirole and/or a pharmaceutically acceptable salt thereof and an adhesive agent, as well as a ropinirole-containing patch with improved holding power.

[Background Art]

Ropinirole is known as a drug useful for treating Parkinson's disease, restless legs syndrome, and the like, and transdermal administration of a preparation containing ropinirole and/or a pharmaceutically acceptable salt thereof has been studied in recent years from the viewpoint of reduction in frequency of administration, improvement in the compliance, and easiness of administration and discontinuation thereof.

For example, International Publication No. WO2010/134433 (PTL 1) states a preparation for transdermal absorption comprising a backing and an adhesive agent layer containing ropinirole or a pharmaceutically acceptable acid addition salt thereof, and International Publication No. WO2012/165253 (PTL 2) states a ropinirole-containing patch comprising a backing layer and an adhesive agent layer containing a specific amount of ropinirole and/or a pharmaceutically acceptable salt thereof.

Moreover, International Publication No. WO2018/155390 (PTL 3) states a patch comprising a backing layer and an adhesive agent layer containing a mixture of a pharmaceutically acceptable acid addition salt of ropinirole and potassium bicarbonate.

In addition, International Publication No. WO2012/165254 (PTL 4) states that free ropinirole plasticizes an adhesive base agent to reduce the cohesive force of an adhesive agent layer. The same document states that in order to achieve excellent skin permeability of ropinirole and sufficient adhesiveness, in a ropinirole-containing patch comprising a backing layer and an adhesive agent layer containing ropinirole and/or a pharmaceutically acceptable salt thereof, the content of ropinirole and/or a pharmaceutically acceptable salt thereof and the mass per unit area of the adhesive agent layer are set within specific ranges, and the adhesive agent layer is caused to contain a specific amount of an alkali metal hydroxide, and also states sodium hydroxide and the like as the alkali metal hydroxide.

[Citation List]

[Patent Literatures]

[PTL 1] International Publication No. WO2010/134433
[PTL 2] International Publication No. WO2012/165253
[PTL 3] International Publication No. WO2018/155390
[PTL 4] International Publication No. WO2012/165254

[Summary of Invention]

[Technical Problem]

Since a patch is used to transdermally administering a drug on a continuous basis by applying an adhesive agent layer to a skin, for example, it is required for the patch to have various physical properties such as adhesive force of the adhesive agent layer to a skin (generally, force indicated by force of peeling off in a direction at 180° or a direction at 90°), cohesive force as described in PTL 4, tackiness (generally, the degree of stickiness indicated by a distance by which a ball is allowed to roll on the surface of an adhesive agent layer set in an incline (ball tack test)), and holding power (power resisting against force of displacement caused by the fluidity of the adhesive agent).

When conducting further studies regarding ropinirole-containing patches, the present inventors have found that in a patch containing ropinirole and/or a pharmaceutically acceptable salt thereof and an adhesive agent in an adhesive agent layer, particularly the holding power tends to decrease among such physical properties.

In addition, in order to improve the holding power, fillers of calcium carbonate and the like as described in PTLs 1 to 4 have conventionally been used. However, the present inventors have found that when these fillers are contained in the adhesive agent layer, there is a case where the skin permeability of ropinirole and/or a pharmaceutically acceptable salt thereof decreases.

The present invention has been made in view of the above problems of the conventional techniques, and an object thereof is to provide a method for improving holding power of an adhesive agent layer in a ropinirole-containing patch that is capable of improving the holding power of an adhesive agent layer containing ropinirole and/or a pharmaceutically acceptable salt thereof and an adhesive agent while maintaining the skin permeability of ropinirole and/or a pharmaceutically acceptable salt thereof, as well as a ropinirole-containing patch with improved holding power that is excellent in holding power of an adhesive agent layer.

[Solution to Problem]

The present inventors continuously conducted earnest studies in order to achieve the above object, and consequently have found that in a ropinirole-containing patch comprising a backing layer and an adhesive agent layer, wherein the adhesive agent layer contains at least one selected from the group consisting of ropinirole and a pharmaceutically acceptable salt thereof and an adhesive agent, surprisingly, it is possible to sufficiently improve holding power of the adhesive agent layer as compared with the case where sodium hydroxide as described in PTL 4 is used, by causing the adhesive agent layer to contain a sodium salt of carbonic acid. Moreover, in contrast to the case where when calcium carbonate or the like, which has conventionally been known as a filler for an adhesive agent layer, is used, the skin permeability of ropinirole and/or a pharmaceutically acceptable salt thereof decreases, the present inventors have found that in the case where the sodium salt of carbonic acid is used, it is possible to sufficiently maintain the skin permeability, and have led to the completion of the present invention.

Specifically, a method for improving holding power of an adhesive agent layer in a ropinirole-containing patch of the present invention is a method for improving holding power of an adhesive agent layer in a ropinirole-containing patch comprising a backing layer and the adhesive agent layer, wherein the adhesive agent layer contains at least one selected from the group consisting of ropinirole and a pharmaceutically acceptable salt thereof and an adhesive agent, the method comprising:
causing the adhesive agent layer to further contain a sodium salt of carbonic acid.

In the method for improving holding power of an adhesive agent layer in a ropinirole-containing patch of the present invention, the adhesive agent is preferably at least one selected from the group consisting of a rubber-based adhesive agent, an acrylic-based adhesive agent having no carboxy group, and a silicone-based adhesive agent.

In addition, in the method for improving holding power of an adhesive agent layer in a ropinirole-containing patch of the present invention, the sodium salt of carbonic acid is preferably at least one selected from the group consisting of sodium carbonate and sodium bicarbonate.

Moreover, the method for improving holding power of an adhesive agent layer in a ropinirole-containing patch of the present invention preferably comprises : causing the adhesive agent layer to contain the sodium salt of carbonic acid such that the content of the sodium salt of carbonic acid relative to a total mass of the adhesive agent layer becomes 0.5 to 33% by mass, and also comprises : causing the adhesive agent layer to contain the sodium salt of carbonic acid such that a content of the sodium salt of carbonic acid relative to 1.0 mol of the at least one selected from the group consisting of ropinirole and a pharmaceutically acceptable salt thereof in content in terms of free ropinirole becomes 0.1 to 5.0 mol.

In addition, in the method for improving holding power of an adhesive agent layer in a ropinirole-containing patch of the present invention, a content of the at least one selected from the group consisting of ropinirole and a pharmaceutically acceptable salt thereof in terms of free ropinirole is preferably 5 to 30% by mass relative to the total mass of the adhesive agent layer.

A ropinirole-containing patch with improved holding power of the present invention is a patch comprising a backing layer and an adhesive agent layer, wherein the adhesive agent layer contains at least one selected from the group consisting of ropinirole and a pharmaceutically acceptable salt thereof, a sodium salt of carbonic acid, and an adhesive agent.

[Advantageous Effects of Invention]

The present invention makes it possible to provide a method for improving holding power of an adhesive agent layer in a ropinirole-containing patch that is capable of improving the holding power of an adhesive agent layer containing ropinirole and/or a pharmaceutically acceptable salt thereof and an adhesive agent while maintaining the skin permeability of ropinirole and/or a pharmaceutically acceptable salt thereof, as well as a ropinirole-containing patch with improved holding power that is excellent in holding power of an adhesive agent layer.

[Description of Embodiments]

Hereinafter, the present invention will be described in detail based on preferred embodiments.

A method for improving holding power of an adhesive agent layer in a ropinirole-containing patch of the present invention (hereinafter, sometimes referred to simply as a " method of the present invention") is a method for improving holding power of an adhesive agent layer in a ropinirole-containing patch comprising a backing layer and an adhesive agent layer, wherein the adhesive agent layer contains at least one selected from the group consisting of ropinirole and a pharmaceutically acceptable salt thereof and an adhesive agent, the method comprising:

causing the adhesive agent layer to further contain a sodium salt of carbonic acid.

The ropinirole-containing patch according to the method of the present invention (hereinafter, sometimes referred to simply as a "patch of the present invention") is a patch comprising a backing layer and an adhesive agent layer, wherein the adhesive agent layer contains, at least, at least one selected from the group consisting of ropinirole and a pharmaceutically acceptable salt thereof and an adhesive agent.

The backing layer according to the present invention is not particularly limited as long as the backing layer can support the adhesive agent layer described later, and a publicly-known backing layer for patches may be employed as appropriate. The material of the backing layer according to the present invention includes, for example, polyolefins such as polyethylene and polypropylene; ethylene-vinyl acetate copolymer, vinyl acetate-vinyl chloride copolymer, polyvinyl chloride, and the like; polyamides such as nylon; polyesters such as polyethylene terephthalate (PET), polybutylene terephthalate, and polyethylene naphthalate; cellulose derivatives; synthetic resins such as polyurethane, and metals such as aluminum. Among these, polyester is preferable from the viewpoint of non-drug adsorbing property and non-drug permeability. The form of the backing layer includes, for example, films; sheets such as a sheet, a sheet-shaped porous body, and a sheet-shaped foam; fabrics such as a woven fabric, a braided fabric, and a nonwoven fabric; foils; and laminates of these. In addition, the thickness of the backing layer is not particularly limited, but is preferably within a range of 5 to 1000 $\mu$m from the viewpoint of easiness of work in applying the patch and easiness of production.

The patch according to the present invention may further comprise a release liner on a surface of the adhesive agent layer opposite to the backing layer. Such a release liner includes a film and a sheet made of a material such as polyolefins such as polyethylene and polypropylene; ethylene-vinyl acetate copolymer, vinyl acetate-vinyl chloride copolymer, polyvinyl chloride, and the like; polyamides such as nylon; polyesters such as polyethylene terephthalate; cellulose derivatives; synthetic resins such as polyurethane, aluminum, and paper, and a laminate of these. These release liners are preferably those processed by a release treatment using a silicone-containing compound coating, fluorine-containing compound coating, or the like on a surface thereof which comes into contact with the adhesive agent layer so that the release liner can be easily peeled off the adhesive agent layer.

The adhesive agent layer according to the present invention contains at least one selected from the group consisting of ropinirole and a pharmaceutically acceptable salt thereof (in the Specification, sometimes referred to as "ropinirole and/or a pharmaceutically acceptable salt thereof") as the drug.

Ropinirole contained in the adhesive agent layer according to the present invention may be a free form (in the Specification, sometimes referred to as "free ropinirole") or may be a pharmaceutically acceptable salt thereof, or a free form obtained by desalting a pharmaceutically acceptable salt of ropinirole in a preparation during the production and/or after the production, and one of these may be used alone or a mixture of two or more of these may be used.

The pharmaceutically acceptable salt of ropinirole is preferably a pharmaceutically acceptable acid addition salt of ropinirole (in the Specification, sometimes referred to as a "ropinirole acid addition salt"). The acid of the ropinirole acid addition salt includes, for example, hydrochloric acid, sulfuric acid, nitric acid, phosphoric acid, phosphorous acid, hydrobromic acid, maleic acid, malic acid, ascorbic acid, tartaric acid, and fumaric acid.

When the adhesive agent layer is caused to contain the ropinirole acid addition salt as an ingredient of the patch according to the present invention, since a sodium salt of carbonic acid described below is also a weak base, when the ropinirole acid addition salt is mixed with the sodium salt of carbonic acid, all or part of the ropinirole acid addition salt is desalted (neutralized) in the adhesive agent layer of the preparation during the production and/or after the production, so that free ropinirole in the state of a free base is obtained, and consequently, it becomes possible to cause free ropinirole having a higher tissue absorption to exist in the adhesive agent layer when the preparation is applied.

The content of the ropinirole and/or a pharmaceutically acceptable salt thereof according to the present invention (the content of free ropinirole or the content of a pharmaceutically acceptable salt of ropinirole, or in a case where both of free ropinirole and a pharmaceutically acceptable salt of ropinirole are contained, the total content of these, the same applies hereinafter.) is preferably 5 to 30% by mass, more preferably 5 to 25% by mass, further preferably 5 to 20% by mass, even more preferably 5 to 15% by mass, and particularly preferably 5 to 13.2% by mass, relative to the total mass of the adhesive agent layer in terms of free ropinirole (in the Specification, the "total mass of the adhesive agent layer" refers to the total mass of the adhesive agent layer in the patch after the manufacture (after a sodium salt of carbonic acid is caused to be contained), and more preferably, a mass obtained by subtracting the mass of the solvent from the total mass of the adhesive agent layer composition described below). If the content of the ropinirole and/or a pharmaceutically acceptable salt thereof is less than the lower limit, the skin permeability of the ropinirole and/or a pharmaceutically acceptable salt thereof (hereinafter, sometimes referred to as "skin permeability of ropinirole") tends to decrease. On the other hand, if the content is more than the upper limit, since the absolute amount in the adhesive agent layer is increased, this tends to make it difficult to spread the composition in the adhesive agent layer when the adhesive agent layer is formed, and make it difficult to obtain a uniform preparation.

The adhesive agent layer according to the present invention contains an adhesive agent. The adhesive agent according to the present invention includes a rubber-based adhesive agent, an acrylic-based adhesive agent, a silicone-based adhesive agent, and the like, and one of these may be used alone or two or more of these may be used in combination. Among these, the adhesive agent according to the present invention is preferably at least one selected from the group consisting of a rubber-based adhesive agent, an acrylic-based adhesive agent having no carboxy group, and a silicone-based adhesive agent, and more preferably contains at least a rubber-based adhesive agent.

The content of the adhesive agent in the adhesive agent layer according to the present invention (which is, in the case where there are two or more, the total content of these, the same applies hereinafter.) is preferably 10 to 90% by mass, preferably 17 to 85% by mass, and further preferably 20 to 70% by mass, relative to the total mass of the adhesive agent layer.

The rubber-based adhesive agent according to the present invention includes natural rubber and synthetic rubbers, and more preferably is at least one selected from the group consisting of synthetic rubbers having no hydroxyl group or carboxy group such as styrene-isoprene-styrene block copolymer (SIS), isoprene rubber, polyisobutylene (PIB), styrene-butadiene-styrene block copolymer (SBS), styrene-butadiene rubber (SBR), and polybutene from the viewpoint that these tend to be excellent in the cohesive force of the adhesive agent layer and the skin permeability of ropinirole. In the present invention, the "synthetic rubbers having no hydroxyl group or carboxy group" represent synthetic rubbers having substantially no hydroxyl group or carboxy group, and preferably those in each of which the content of the hydroxyl group and the carboxy group in the molecule is less than 3% by mass.

In the case where the adhesive agent layer according to the present invention contains the rubber-based adhesive agent, the content of the rubber-based adhesive agent (which is, in the case where there are two or more, the total content of these, the same applies hereinafter.) is preferably 10 to 55% by mass, and more preferably 15 to 35% by mass, relative to the total mass of the adhesive agent layer. If the content of the rubber-based adhesive agent is less than the lower limit, the cohesive force of the adhesive agent layer tends to decrease. On the other hand, if the content is more than the upper limit, the adhesive agent layer becomes so hard that the adhesive force of the patch tends to decrease.

The acrylic-based adhesive agent according to the present invention includes those listed as adhesive agents in "Japanese Pharmaceutical Excipients Directory 2016 (edited by International Pharmaceutical Excipients Council Japan)". One of these may be used alone or two or more of these may be used in combination, but the acrylic-based adhesive agent is preferably an acrylic-based adhesive agent having no carboxy group, and more preferably an acrylic-based adhesive agent having no functional group. In the present invention, the "acrylic-based adhesive agent having no carboxy group" and the "acrylic-based adhesive agent having no functional group" represent acrylic-based polymers having substantially no carboxy group or functional group, and preferably those in each of which the content of the carboxy group and the functional group (including the carboxy group) in the polymer is less than 3% by mass.

The acrylic-based adhesive agent having no carboxy group includes, for example, acrylic-based adhesive agents that have substantially no functional group such as 2-ethylhexyl acrylate·vinylpyrrolidone copolymer, 2-ethylhexyl acrylate·2-ethylhexyl methacrylate·dodecyl methacrylate copolymer, 2-ethylhexyl acrylate·vinyl acetate copolymer, 2-ethylhexyl acrylate·methyl methacrylate·butyl acrylate copolymer, 2-ethylhexyl acrylate·methacrylic acid copolymer, and ethyl acrylate·methyl methacrylate copolymer; and acrylic-based adhesive agents that have a hydroxy group such as 2-ethylhexyl (meth)acrylate·vinyl acetate·2-hydroxyethyl acrylate copolymer, 2-hydroxyethyl (meth)acrylate copolymer, 2-hydroxypropyl (meth)acrylate copolymer, 3-hydroxypropyl (meth)acrylate copolymer, 4-hydroxybutyl (meth)acrylate copolymer, and 2-ethylhexyl acrylate·vinyl acetate·hydroxyethyl acrylate·glycidyl methacrylate copolymer. One of these may be used alone or two or more of these may be used in combination.

As the acrylic-based adhesive agent having no carboxy group, a commercially-available one may be used as appropriate, and it is possible to use as appropriate, for example, acrylic-based polymers contained in MAS 811 and MAS 683 (manufactured by CosMED Pharmaceutical Co. Ltd.); 87-900A, 87-901A, 87-9301, 87-4098, 87-9088, and 87-9085 of Duro-Tak (registered trademark) acrylic adhesive agent series (manufactured by Henkel AG & Co. KGaA); GMS 3083, GMS 3253, and GMS 3235 of GELVA (registered trademark) acrylic adhesive agent series (manufactured by Henkel AG & Co. KGaA), and the like, and acrylic-based polymers contained in 87-202A, 87-2287, 87-2516, 87-2510, 87-4287, 87-2525, 87-201A, 87-202A, 87-208A, 87-502A, 87-503A, and 87-504A of Duro-Tak (registered trademark) acrylic adhesive agent series (manufactured by Henkel AG & Co. KGaA); GMS 788 and GMS 737 of GELVA (registered trademark) acrylic adhesive agent series (manufactured by Henkel AG & Co. KGaA), and the like.

In the case where the adhesive agent layer according to the present invention contains an acrylic-based adhesive agent, the content of the acrylic-based adhesive agent (which is, in the case where there are two or more, the total content of these, the same applies hereinafter.) is preferably 10 to 90% by mass, more preferably 20 to 85% by mass, and further preferably 20 to 70% by mass, relative to the total mass of the adhesive agent layer. If the content of the acrylic-based adhesive agent is less than the lower limit, the cohesive force of the adhesive agent layer tends to decrease. On the other hand, if the content is more than the upper limit, the skin permeability of ropinirole tends to decrease.

In the case where the adhesive agent layer according to the present invention contains a silicone-based adhesive agent, the silicone-based adhesive agent includes silicone rubbers represented by MQ (polydimethylsiloxane), VMQ (polymethylvinylsiloxane), PMQ (polymethylphenylsiloxane), and PVMQ (polyphenylvinylmethylsiloxane) in ASTM standard (ASTM D 1418), a mixture of at least one of these and a silicone resin such as polyditrimethylsilylsiloxane other than silicone rubbers, and the like. One of these may be used alone or two or more of these may be used in combination. Note that in the case where a silicone resin other than the silicone rubbers is mixed, the content of the silicone resin is preferably 0.1 to 20% by mass relative to the total mass of the silicone-based adhesive agent.

In addition, as these silicone-based adhesive agents, commercially-available ones may be used as appropriate. For example, silicone-based adhesive agents provided by DuPont Toray Specialty Materials K.K. under the following model numbers: BIO-PSA7-410X, BIO-PSA7-420X, BIO-PSA7-430X, BIO-PSA7-440X, BIO-PSA7-450X, BIO-PSA7-460X (the Xs are each independently 1 or 2), BIO-PSA AC7-4201, BIO-PSA AC7-4301, BIO-PSA AC7-4302, MD7-4502, MD7-4602, 7-9700, MG7-9800, MG7-9850; BIO-PSA 7-4560, which is a hot melt silicone adhesive agent, and the like may be used as appropriate.

Moreover, the silicone-based adhesive agent according to the present invention may be, for example, in the case of having methyl groups, one obtained by dehydrogenating the methyl groups to remove hydrogen atoms to crosslink the methyl groups by blending a peroxide; in the case of having vinyl groups, one obtained by binding a crosslinking agent composed of a SiH group-containing siloxane compound to crosslink the vinyl groups; in the case of having hydroxyl groups (that is, in the case of having silanol groups), one obtained by crosslinking the silanol groups through dehydration condensation, or the like.

In the case where the adhesive agent layer according to the present invention contains the silicone-based adhesive agent, the content of the silicone-based adhesive agent (which is, in the case where there are two or more, the total content of these, the same applies hereinafter.) is preferably 10 to 90% by mass, more preferably 20 to 85% by mass, and further preferably 20 to 70% by mass relative to the total mass of the adhesive agent layer. If the content of the silicone-based adhesive agent is less than the lower limit, the cohesive force of the adhesive agent layer tends to decrease. On the other hand, if the content is more than the upper limit, the skin permeability of ropinirole tends to decrease.

The adhesive agent layer according to the present invention may further contain another drug other than ropinirole and a pharmaceutically acceptable salt thereof; an absorption enhancer (transdermal absorption enhancer) ; an excipient such as an adsorbent, a tackifier, a plasticizer, a solubilizer, a filler, a stabilizer, a preservative, or the like as long as the effect of the present invention is not hindered.

The other drug other than ropinirole and a pharmaceutically acceptable salt thereof includes, for example, non-steroidal anti-inflammatory drugs (diclofenac, indomethacin, ketoprofen, felbinac, loxoprofen, ibuprofen, flurbiprofen, tiaprofenic acid, acemetacin, sulindac, etodolac, tolmetin, piroxicam, meloxicam, ampiroxicam, naproxen, azapropazone, methyl salicylate, glycol salicylate, valdecoxib, celecoxib, rofecoxib, amfenac, and the like), anti-histamine drugs (diphenhydramine, chlorpheniramine, mequitazine, homochlorcyclizine, and the like), anti-hypertensive drugs (diltiazem, nicardipine, nilvadipine, metoprolol, bisoprolol, trandolapril, and the like), anti-Parkinson drugs (pergolide, bromocriptine, selegiline, and the like), bronchodilators (tulobuterol, isoproterenol, salbutamol, and the like), anti-allergic drugs (ketotifen, loratadine, azelastine, terfenadine, cetirizine, acitazanolast, and the like), local anesthetics (lidocaine, dibucaine, and the like), anesthetic-based analgesics (morphine and the like), agents for urinary organs (oxybutynin, tamsulosin, and the like), neuropsychiatric drugs (promazine, chlorpromazine, and the like), steroid hormone drugs (estradiol, progesterone, norethisterone, cortisone, hydrocortisone, and the like), antidepressants (sertraline, fluoxetine, paroxetine, citalopram, and the like), anti-dementia drugs (donepezil, rivastigmine, galantamine, and the like), anti-psychotic drugs (risperidone, olanzapine, and the like), central nervous system stimulant drugs (methylphenidate and the like), osteoporosis treatment drugs (raloxifene, alendronate, and the like), breast cancer preventive drugs (tamoxifen and the like), anti-obesity drugs (mazindol, sibutramine, and the like), insomnia treatment drugs (melatonin and the like), and anti-rheumatic drugs (actarit and the like). One of these may be used alone or two or more of these may be used in combination.

The absorption enhancer includes, for example, isopropyl myristate, isopropyl palmitate, lauryl alcohol, hexyl laurate, myristyl alcohol, oleyl alcohol, isostearyl alcohol, octyldodecanol, benzyl alcohol, glycerine monooleate (GMO), propylene glycol monolaurate (PGML), polyoxyethylene sorbitan monooleate (Tween 80), polyoxyethylene sorbitan tristearate (Tween 65), polyoxyethylene sorbitan monostearate (Tween 60), polyoxyethylene sorbitan monolaurate (Tween 20), lauric acid diethanolamide (LADA), and the like. One of these may be used alone or two or more of these may be used in combination.

The adsorbent includes inorganic and/or organic substances having moisture absorbency, and more specifically includes minerals such as talc, kaolin, and bentonite; silicon compounds such as fumed silica (Aerosil (registered trademark) and the like) and hydrous silica; metal compounds such as zinc oxide and dried aluminum hydroxide gel; weak acids such as lactic acid and acetic acid; sugars such as dextrin; and high molecular polymers such as polyvinylpyrrolidone, aminoalkyl methacrylate copolymer, crospovidone, carboxy vinyl polymer, and butyl methacrylate-methyl methacrylate copolymer. One of these may be used alone or two or more of these may be used in combination.

The tackifier is blended mainly for the purpose of enhancing the adhesive force of the adhesive agent. Such a tackifier includes, for example, rosin-based resins, terpene-based resins, petroleum-based resins (alicyclic saturated hydrocarbon resin and the like), phenolic resins, and xylene-based resins. One of these may be used alone or two or more of these may be used in combination. In the case where such a tackifier is further contained in the adhesive agent layer, the content of the tackifier (which is, in the case where there are two or more, the total content of these) is more preferably 10 to 80% by mass, and further preferably 20 to 60% by mass, relative to the total mass of the adhesive agent layer from the viewpoint of improving the adhesive force of the adhesive agent layer and/or alleviating local irritation at the peeling-off.

The plasticizer is blended mainly for the purpose of adjusting the adhesiveness of the adhesive agent layer, the fluidity in the production of the adhesive agent layer, the transdermal absorption feature of the drug, and the like. Such a plasticizer includes, for example, silicone oil; petroleum-based oils such as paraffinic process oils such as liquid paraffin, naphthenic process oils, and aromatic process oils; squalane, squalene; vegetable-based oils such as olive oil, camellia oil, castor oil, tall oil, and peanut oil; dibasic esters such as dibutyl phthalate and dioctyl phthalate; liquid rubbers such as liquid polybutene and liquid isoprene rubber; diethylene glycol, polyethylene glycol, propylene glycol, dipropylene glycol, and the like. One of these may be used alone or two or more of these may be used in combination. Among these, the plasticizer is preferably at least one selected from the group consisting of silicone oil, liquid paraffin, and liquid polybutene. In the case where the plasticizer is further contained in the adhesive agent layer, the content of the plasticizer (which is, in the case where there are two or more, the total content of these) is more preferably 5 to 60% by mass, and further preferably 7 to 40% by mass, relative to the total mass of the adhesive agent layer from the viewpoint that the adhesive force as the patch becomes more favorable.

The solubilizer is blended mainly for the purpose of facilitating the dissolution of the drug. Such a solubilizer includes, for example, organic acids such as acetic acid, aliphatic alcohols, and surfactants. One of these may be used alone or two or more of these may be used in combination. Among these, the solubilizer is preferably at least one selected from the group consisting of organic acids and aliphatic alcohols.

The filler is blended mainly for the purpose of adjusting the adhesive force and the holding power of the adhesive agent layer. Such a filler includes, for example, aluminum hydroxide, calcium carbonate, magnesium carbonate; silicates such as aluminum silicate and magnesium silicate; silicic acid, barium sulfate, calcium sulfate, calcium zincate, zinc oxide, and titanium oxide. One of these may be used alone or two or more of these may be used in combination. Since according to the method of the present invention, a patch excellent in holding power of the adhesive agent layer can be obtained even without blending these fillers, such a filler substantially does not have to be blended also from the viewpoint of maintaining the skin permeability of ropinirole. In this case, more specifically, the amount of the filler blended (which is, in the case where there are two or more, the total amount of these blended) is preferably 1% by mass or less, and more preferably 0.1% by mass or less, relative to the total mass of the adhesive agent layer.

For the adhesive agent layer according to the present invention, a component that functions as a desalting agent for the pharmaceutically acceptable acid addition salt of ropinirole besides the sodium salt of carbonic acid described below may further be used as long as the effect of the present invention is not hindered. Such a component includes, for example, metal ion-containing desalting agents and basic nitrogen atom-containing desalting agents. The metal ion-containing desalting agent includes strong bases such as sodium hydroxide and potassium hydroxide. One of these may be used alone or two or more of these may be used in combination. In addition, weak bases such as sodium acetate (including anhydrous sodium acetate), magnesium hydroxide, sodium citrate, and sodium lactate are also components that can function as desalting agents. Since according to the method of the present invention, the patch is excellent in skin permeability of ropinirole even without blending these components, such a component substantially does not have to be blended. In this case, more specifically, the amount of the component blended (which is, in the case where there are two or more, the total amount of these blended) is preferably 1% by mass or less, and more preferably 0.1% by mass or less, relative to the total mass of the adhesive agent layer.

The thickness of the adhesive agent layer according to the present invention (the thickness after the production (after the sodium salt of carbonic acid is caused to be contained), the same applies hereinafter.) is not particularly limited, but is preferably a thickness with which the mass per unit area of the adhesive agent layer becomes 25 to 250 $g/m^2$, more preferably a thickness with which the mass per unit area becomes 50 to 200 $g/m^2$, further preferably a thickness with which the mass per unit area becomes 50 to 150 $g/m^2$, and even more preferably a thickness with which the mass per unit area becomes 50 to 120 $g/m^2$, for example. The area to apply the patch according to the present invention is preferably 1 to 300 $cm^2$, and more preferably 5 to 30 $cm^2$.

In the method of the present invention, the adhesive agent layer containing the ropinirole and/or a pharmaceutically acceptable salt thereof and the adhesive agent is caused to contain a sodium salt of carbonic acid. In the present invention, the "sodium salt of carbonic acid" refers to a compound that is a salt composed of an acid selected from the group consisting of carbonic acid and hydrogencarbonate and sodium, in which a carbonate ion ($CO_3^{2-}$) or hydrogen-carbonate ion ($HCO_3^-$) and a sodium ion ($Na^+$) are bonded to each other. More specifically, the sodium salt of carbonic acid according to the present invention includes sodium carbonate and sodium bicarbonate, and may be one of these alone or two or more of these in combination.

In the method of the present invention, the adhesive agent layer is preferably caused to contain the sodium salt of carbonic acid such that the content of the sodium salt of carbonic acid (which is, in the case where there are two or more, the total content of these, the same applies hereinafter.) relative to the total mass of the adhesive agent layer becomes 0.5 to 33% by mass, is more preferably caused to contain the sodium salt of carbonic acid such that the content of the sodium salt of carbonic acid becomes 0.5 to 25% by mass, and is further preferably caused to contain the sodium salt of carbonic acid such that the content of the sodium salt of carbonic acid becomes 1 to 20% by mass. If the content of the sodium salt of carbonic acid in the obtained adhesive agent layer is less than the lower limit, the effect to improve the holding power of the adhesive agent layer tends not to be sufficiently exhibited. On the other hand, the content is more than the upper limit, the cohesive force of the adhesive agent layer tends to decrease. Note that, in a case where a component derived from a sodium salt of carbonic acid described below is contained in addition to the content of the sodium salt of carbonic acid, the content of the sodium salt of carbonic acid according to the present invention also contains the content of the component in terms of the sodium salt of carbonic acid.

In addition, in the method of the present invention, the adhesive agent layer is preferably caused to contain the sodium salt of carbonic acid such that the content of the sodium salt of carbonic acid relative to 1.0 mol of the ropinirole and/or a pharmaceutically acceptable salt thereof in content in terms of free ropinirole becomes 0.1 to 5.0 mol in terms of carbonic acid or hydrogencarbonate (in terms of the number of $CO_3$ or $HCO_3$). The adhesive agent layer is more preferably caused to contain the sodium salt of carbonic acid such that the content of the sodium salt of carbonic acid becomes 0.5 to 5.0 mol, and is further preferably caused to contain the sodium salt of carbonic acid such that the content of the sodium salt of carbonic acid becomes 1.0 to 5.0 mol, from the viewpoint of a balance between the maintenance of a sufficient skin permeability of ropinirole and the effect to improve the holding power of the adhesive agent layer. Moreover, the adhesive agent layer is more preferably caused to contain the sodium salt of carbonic acid such that the content of the sodium salt of carbonic acid becomes 0.2 to 4.0 mol, is further preferably caused to contain the sodium salt of carbonic acid such that the content of the sodium salt of carbonic acid becomes 0.5 to 3.0 mol, and is even more preferably caused to contain the sodium salt of carbonic acid such that the content of the sodium salt of carbonic acid becomes 1.0 to 2.0 mol, from the viewpoint of further sufficiently maintaining the cohesive force of the adhesive agent layer. If the content of the sodium salt of carbonic acid in the obtained adhesive agent layer is less than the lower limit, the skin permeability of the ropinirole tends to decrease. On the other hand, if the content is more than the upper limit, the cohesive force of the adhesive agent layer tends to decrease.

The method for causing the adhesive agent layer to further contain the sodium salt of carbonic acid according to the present invention includes, for example, a method comprising: mixing the ropinirole and/or a pharmaceutically acceptable salt thereof, the sodium salt of carbonic acid, and the adhesive agent; and molding an adhesive agent layer composition thus obtained to obtain the adhesive agent layer in a method for producing a ropinirole-containing patch.

More specifically, the method for producing the ropinirole-containing patch includes, for example, the following production method. First, the ropinirole and/or a pharmaceutically acceptable salt thereof, the sodium salt of carbonic acid, and the adhesive agent, as well as, the other drug, the absorption enhancer, the additive, the solvent, and the like as necessary, are mixed in accordance with a conventional method to obtain a uniform adhesive agent layer composition. The solvent includes, for example, water, anhydrous ethanol, toluene, hexane, ethyl acetate, cyclohexane, heptane, butyl acetate, ethanol, methanol, xylene, isopropanol, a mixture liquid of two or more of these, and the like. Subsequently, this adhesive agent layer composition is spread onto a surface of the backing layer (normally one of the surfaces), and thereafter, the solvent is dried and removed as necessary to form an adhesive agent layer. The product is further cut into a desired shape as necessary.

In addition, the method for producing the ropinirole-containing patch may further include a step of applying the release liner on a surface of the adhesive agent layer opposite to the backing layer. The production method may be such that after the adhesive agent layer composition is first spread onto one surface of the release liner to form the adhesive agent layer, the backing layer is applied onto the surface of the adhesive agent layer opposite to the release liner, and the product is cut into a desired shape as necessary to obtain a patch.

In this way, in the ropinirole-containing patch thus obtained, it is possible to sufficiently improve the holding power of the adhesive agent layer without lowering the skin permeability of ropinirole and/or a pharmaceutically acceptable salt thereof. Hence, as an improved patch with improved holding power of an adhesive agent layer, the present invention also provides a ropinirole-containing patch with improved holding power comprising the backing layer and the adhesive agent layer, wherein the adhesive agent layer contains the ropinirole and/or a pharmaceutically acceptable salt thereof, the sodium salt of carbonic acid, and the adhesive agent.

In the ropinirole-containing patch with improved holding power of the present invention, the backing layer, the adhesive agent layer, the ropinirole and/or a pharmaceutically acceptable salt thereof, the sodium salt of carbonic acid, and the adhesive agent correspond to those of the above ropinirole-containing patch according to the method of the present invention, including their preferable aspects and preferable contents.

In a case where the adhesive agent layer is caused to contain the ropinirole acid addition salt as an ingredient of a patch, all or part of the ropinirole acid addition salt is desalted in the adhesive agent layer of the preparation during the production and/or after the production by the sodium salt of carbonic acid, which is also a weak base. Hence, the adhesive agent layer in the ropinirole-containing patch with improved holding power of the present invention obtained in this case only has to contain at least a mixture of the ropinirole acid addition salt and the sodium salt of carbonic acid besides the adhesive agent, and may contain, as the mixture, a component derived from free ropinirole and the sodium salt of carbonic acid (component derived from the sodium salt of carbonic acid) as a desalting reaction product of the ropinirole acid addition salt and the sodium salt of carbonic acid.

The component derived from the sodium salt of carbonic acid includes carbonic acid, carbonate ions, hydrogencarbonate, hydrogencarbonate ions, sodium ions, and sodium salts, and one of these may be used alone or two or more of these may be used in combination. Although it depends on the acid of the ropinirole acid addition salt or the type of the acid which may be contained in the adhesive agent layer as necessary, the sodium salt includes, for example, sodium salts of acids such as hydrochloric acid, sulfuric acid, nitric acid, phosphoric acid, phosphorous acid, hydrobromic acid, maleic acid, malic acid, ascorbic acid, tartaric acid, and fumaric acid.

Such a ropinirole-containing patch with improved holding power may be packaged (preferably sealed) in a package container for a period after the production to the time of use. The package container is not particularly limited, and a package container that can normally be used as a package container for patches may be used as appropriate. For example, it is preferable to use a plastic packaging bag, a plastic packaging bag in which a metal layer (for example, an aluminum layer) is formed, a metal packaging bag (for example, an aluminum packaging bag), and the like.

In addition, the packaged formulation in which the ropinirole-containing patch with improved holding power is packaged in the package container may further have oxygen absorbing means. The oxygen absorbing means includes an oxygen absorber using an iron powder and an oxygen absorber containing vitamin C as a main component which are to be sealed in the package container (more specifically, AGELESS series (manufactured by Mitsubishi Gas Chemical Company, Inc.), PharmaKeep series (manufactured by Mitsubishi Gas Chemical Company, Inc.), and the like) ; and the package container including a layer having an oxygen absorbing function (more specifically, a layer in which a powder of aluminum, zinc, manganese, copper, iron, hydrosulfite, activated carbon, or the like is mixed, or the like).

[Examples]

The present invention will be described more specifically below based on Examples and Comparative Examples; however, the present invention is not limited to Examples below. Note that a skin permeation test and a holding power test on patches obtained respectively in Examples and Comparative Examples were performed by methods described below.

<Skin Permeation Test (In Vitro Hairless Mouse Skin Permeation Test)>

First, the skin of the trunk of a hairless mouse was peeled off and the adipose was removed, and the patch which had been cut into the size of 3.14 cm$^2$ and from which the release liner had been removed was applied to the epidermis side. This was set in a flow through-type Franz permeation test cell such that the dermis side came into contact with a receptor solution, and the cell was filled with the receptor solution (PBS). Subsequently, the receptor solution was sent at a flow rate of about 2.5 mL/hr while warmed circulation water was caused to circulate on the outer periphery such that the receptor solution was kept at 32°C. Then, the receptor solution was sampled every 4 hours until 24 hours. The concentration of ropinirole (ropinirole hydrochloride) in the sampled receptor solution was measured by a high performance liquid chromatography, and the skin permeation amount of ropinirole per unit area of the adhesive agent layer was calculated in accordance with the following formula:

$$\text{the skin permeation amount of ropinirole } (\mu g/cm^2) = \{\text{the concentration of ropinirole in the receptor solution } (\mu g/mL) \times \text{the flow rate (mL)}\}/\text{the patch area } (cm^2).$$

The cumulative skin permeation amount of ropinirole for 24 hours after the start of the application was obtained as a 24 hr cumulative skin permeation amount ($\mu g/cm^2$).

<Holding Power Test>

First, each patch was cut into the size of a width of 10 mm and a length of 50 mm to fabricate a test piece. The release liner was removed from each test piece to expose the surface of the adhesive agent layer, was placed in a center of one end of a stainless steel plate (formed of SUS304), which was a test plate, such that the area to apply was a width of 10 mm and a length of 25 mm, and was pressed by reciprocating a roller of 1 kg one turn to apply the test piece onto the test plate. Immediately after the pressing with the roller, the test plate was hung such that the direction of the length of the test piece became the vertical direction. A weight of 500 g was attached to the free end (lower end) of the test piece, and an elapse of time from when the weight was attached to when the test piece was peeled off from the test plate was measured under an environment of 32°C and 65%RH. In this holding power test, conditions other than those described above were in accordance with the method described in JIS standards (JIS-Z-0237: 2009).

(Example 1)

First, 15.0 parts by mass of ropinirole hydrochloride, 4.2 parts by mass of sodium bicarbonate (corresponding to 1.0 mol relative to 1.0 mol of ropinirole hydrochloride in number of moles), 13.4 parts by mass of styrene-isoprene-styrene block copolymer (SIS), 9.0 parts by mass of polyisobutylene (PIB), 40.4 parts by mass of alicyclic saturated hydrocarbon resin, and 18.0 parts by mass of liquid paraffin were added to an appropriate amount of a solvent (water, methanol, and toluene), followed by mixing to obtain an adhesive agent layer composition. Subsequently, the adhesive agent layer composition thus obtained was spread onto a release liner (a film made of polyester and processed by release treatment) to have a thickness of 100 g/m$^2$, and the solvent was dried and removed to form an adhesive agent layer. A backing layer (a film made of polyester) was laminated on a surface of the adhesive agent layer thus obtained opposite to the release liner to obtain a patch in which the backing layer/the adhesive agent layer/the release liner were laminated in this order.

(Comparative Examples 1 to 2)

Patches were obtained in the same manner as in Example 1 except that the composition of the adhesive agent layer composition became compositions shown in Table 1 below.

The above-described skin permeation test and holding power test were conducted on each of the patches obtained in Example 1 and Comparative Examples 1 to 2. The results are shown in Table 1 below together with the compositions (except for the solvent) of the respective adhesive agent layer compositions. Note that in Table 1, the numerical value in parentheses in the column of ropinirole hydrochloride indicates parts by mass in terms of free ropinirole (the same applies to Tables 2 to 3 below).

[Table 1]

| | Comparative Example 1 | Example 1 | Comparative Example 2 |
|---|---|---|---|
| Adhesive agent layer composition [parts by mass] | | | |
| Ropinirole hydrochloride (in terms of free form) | 15.0 (13.2) | 15.0 (13.2) | 15.0 (13.2) |
| Sodium hydroxide | 2.0 | - | - |
| Sodium bicarbonate | - | 4.2 | - |
| Calcium carbonate | - | - | 5.1 |
| SIS | 13.9 | 13.4 | 13.2 |
| PIB | 9.2 | 9.0 | 8.9 |
| Alicyclic saturated hydrocarbon resin | 41.5 | 40.4 | 40.0 |
| Liquid paraffin | 18.4 | 18.0 | 17.8 |
| Total | 100 | 100 | 100 |
| 24 Hr cumulative skin permeation amount [μg/cm$^2$] | 411 | 366 | 20 |
| Holding power [sec] | 8.50 | 25.00 | 20.50 |

As is clear from the results shown in Table 1, it was confirmed that the patch in which the adhesive agent layer was caused to contain a sodium salt of carbonic acid according to the present invention (for example, Example 1) was excellent in skin permeability of ropinirole as in the case of the patch in which the adhesive agent layer was caused to contain sodium hydroxide (for example, Comparative Example 1). On the other hand, it was confirmed that in the patch in which the adhesive agent layer was caused to contain calcium carbonate, which was a carbonate like a sodium salt of carbonic acid and has been known as a filler (for example, Comparative Example 2), the skin permeability of the ropinirole significantly decreased.

Moreover, as is clear from the results shown in Table 1, in the patch in which the adhesive agent layer was caused to contain the sodium salt of carbonic acid according to the present invention (for example, Example 1), the holding power was significantly improved as compared with the patch in which the adhesive agent layer was caused to contain sodium hydroxide (for example, Comparative Example 1), and also the improving effect was similar to or more excellent than that of the patch in which the adhesive agent layer was caused to contain calcium carbonate (for example, Comparative Example 2) .

(Examples 2 to 3)

Patches were obtained in the same manner as in Example 1 except that the composition of the adhesive agent layer composition became compositions shown in Table 2.

The above-described skin permeation test and holding power test were conducted on each of the patches obtained in Examples 2 to 3. The results are shown in Table 2 below together with the compositions (except for the solvent) of the respective adhesive agent layer compositions. In addition, the results of Comparative Example 1 and Example 1 are also shown together in Table 2. Note that in Table 2, the numerical values in parentheses in the columns of sodium hydroxide and sodium bicarbonate each indicate the number of moles of the content relative to the content of the ropinirole hydrochloride when the content of the ropinirole hydrochloride in terms of free form was regarded as 1.0 mol.

[Table 2]

| | Comparative Example 1 | Example 1 | Example 2 | Example 3 |
|---|---|---|---|---|
| Adhesive agent layer composition [parts by mass] | | | | |
| Ropinirole hydrochloride (in terms of free form) | 15.0 (13.2) | 15.0 (13.2) | 15.0 (13.2) | 15.0 (13.2) |
| Sodium hydroxide (Mol relative to 1.0 mol of free ropinirole) | 2.0 (1.0) | - | - | - |
| Sodium bicarbonate (Mol relative to 1.0 mol of free ropinirole) | - | 4.2 (1.0) | 12.6 (3.0) | 21.0 (5.0) |
| SIS | 13.9 | 13.4 | 12.1 | 10.7 |
| PIB | 9.2 | 9.0 | 8.0 | 7.1 |
| Alicyclic saturated hydrocarbon resin | 41.5 | 40.4 | 36.2 | 32.0 |
| Liquid paraffin | 18.4 | 18.0 | 16.1 | 14.2 |
| Total | 100 | 100 | 100 | 100 |
| 24 Hr cumulative skin permeation amount [$\mu$g/cm$^2$] | 411 | 366 | 569 | 509 |
| Holding power [sec] | 8.50 | 25.00 | 65.50 | 26.00 |

As is clear from the results shown in Table 2, it was confirmed that in the patch in which the adhesive agent layer was caused to contain a sodium salt of carbonic acid according to the present invention, it was possible to improve the holding power of the adhesive agent layer while maintaining a sufficient skin permeability of ropinirole even when the number of moles relative to 1.0 mol of free ropinirole was 3.0 mol or 5.0 mol (for example, Example 2 to 3) like when the number of moles was 1.0 mol (for example, Example 1).

(Examples 4 to 5, Comparative Examples 3 to 4)

Patches were obtained in the same manner as in Example 1 except that the composition of the adhesive agent layer composition became compositions shown in Table 3 below. In Table 3, each adhesive agent is as follows:

MAS-811C: an acrylic-based adhesive agent having substantially no functional group (manufactured by CosMED Pharmaceutical Co. Ltd.), and

Bio-PSA-7-4202: Bio-PSA-7-4202, a silicone-based adhesive agent (manufactured by DuPont Toray Specialty Materials K.K.).

The above-described skin permeation test and holding power test were conducted on each of the patches obtained in Examples 4 to 5 and Comparative Examples 3 to 4. The results are shown in Table 3 below together with the compositions (except for the solvent) of the respective adhesive agent layer compositions. Note that in Table 3, the holding powers of Examples 4 and 5 are relative values when the holding powers of Comparative Examples 3 and 4 are regarded as 1.

[Table 3]

|  | Comparative Example 3 | Example 4 | Comparative Example 4 | Example 5 |
|---|---|---|---|---|
| Adhesive agent layer composition [parts by mass] |  |  |  |  |
| Ropinirole hydrochloride (in terms of free form) | 15.0 (13.2) | 15.0 (13.2) | 15.0 (13.2) | 15.0 (13.2) |
| Sodium hydroxide | 2.0 | - | 2.0 | - |
| Sodium bicarbonate | - | 4.2 | - | 4.2 |
| MAS-811C | 83.0 | 80.8 | - | - |
| BiO-PSA-7-4202 | - | - | 83.0 | 80.8 |
| Total | 100 | 100 | 100 | 100 |
| 24 Hr cumulative skin permeation amount [$\mu$g/cm$^2$] | 659 | 401 | 383 | 548 |
| Holding power [relative to Comparative Example] | 1 | 9.8 | 1 | 83 |

As is clear from the results shown in Table 3, it was confirmed that in the patch in which the adhesive agent layer was caused to contain a sodium salt of carbonic acid according to the present invention (for example, Examples 4 and 5), it was possible to improve the holding power of the adhesive agent layer while maintaining a sufficient skin permeability of ropinirole even when an acrylic-based adhesive agent or a silicone-based adhesive agent was used in placed of rubber-based adhesive agent (for example, SIS and PIB) as an adhesive agent.

From these results, it was confirmed that it was possible to sufficiently improve the holding power of an adhesive agent layer while maintaining a sufficient skin permeability of ropinirole and/or a pharmaceutically acceptable salt thereof by causing the adhesive agent layer containing ropinirole and/or a pharmaceutically acceptable salt thereof and an adhesive agent to further contain a sodium salt of carbonic acid.

[Industrial Applicability]

As described above, the present invention makes it possible to provide a method for improving holding power of an adhesive agent layer in a ropinirole-containing patch that is capable of improving the holding power of an adhesive agent layer containing ropinirole and/or a pharmaceutically acceptable salt thereof and an adhesive agent while maintaining a sufficient skin permeability of ropinirole and/or a pharmaceutically acceptable salt thereof, as well as a ropinirole-containing patch with improved holding power that is excellent in holding power of an adhesive agent layer.

**Claims**

1. A method for improving holding power of an adhesive agent layer in a ropinirole-containing patch comprising a backing layer and the adhesive agent layer, wherein the adhesive agent layer contains at least one selected from the group consisting of ropinirole and a pharmaceutically acceptable salt thereof and an adhesive agent, the method comprising:
causing the adhesive agent layer to further contain a sodium salt of carbonic acid.

2. The method for improving holding power of an adhesive agent layer in a ropinirole-containing patch according to claim 1, wherein
the adhesive agent is at least one selected from the group consisting of a rubber-based adhesive agent, an acrylic-based adhesive agent having no carboxy group, and a silicone-based adhesive agent.

3. The method for improving holding power of an adhesive agent layer in a ropinirole-containing patch according to claim 1 or 2, wherein
the sodium salt of carbonic acid is at least one selected from the group consisting of sodium carbonate and sodium bicarbonate.

4. The method for improving holding power of an adhesive agent layer in a ropinirole-containing patch according to any one of claims 1 to 3, comprising:
causing the adhesive agent layer to contain the sodium salt of carbonic acid such that a content of the sodium salt of carbonic acid relative to a total mass of the adhesive agent layer becomes 0.5 to 33% by mass.

5. The method for improving holding power of an adhesive agent layer in a ropinirole-containing patch according to any one of claims 1 to 4, comprising:
causing the adhesive agent layer to contain the sodium salt of carbonic acid such that a content of the sodium salt of carbonic acid relative to 1.0 mol of the at least one selected from the group consisting of ropinirole and a pharmaceutically acceptable salt thereof in content in terms of free ropinirole becomes 0.1 to 5.0 mol.

6. The method for improving holding power of an adhesive agent layer in a ropinirole-containing patch according to any one of claims 1 to 5, wherein
a content of the at least one selected from the group consisting of ropinirole and a pharmaceutically acceptable salt thereof in terms of free ropinirole is 5 to 30% by mass relative to the total mass of the adhesive agent layer.

7. A ropinirole-containing patch with improved holding power comprising a backing layer and an adhesive agent layer, wherein
the adhesive agent layer contains at least one selected from the group consisting of ropinirole and a pharmaceutically acceptable salt thereof, a sodium salt of carbonic acid, and an adhesive agent.

## INTERNATIONAL SEARCH REPORT

| International application No. |
| --- |
| PCT/JP2020/043382 |

**A. CLASSIFICATION OF SUBJECT MATTER**
A61K 31/4045(2006.01)i; A61K 9/70(2006.01)i; A61K 47/02(2006.01)i; A61K 47/32(2006.01)i; A61K 47/34(2017.01)i; A61P 25/00(2006.01)n; A61P 25/16(2006.01)n
FI:     A61K31/4045; A61K9/70 401; A61K47/02; A61K47/32; A61K47/34; A61P25/00; A61P25/16
According to International Patent Classification (IPC) or to both national classification and IPC

**B. FIELDS SEARCHED**

Minimum documentation searched (classification system followed by classification symbols)
A61K31/4045; A61K9/70; A61K47/02; A61K47/32; A61K47/34; A61P25/00; A61P25/16

Documentation searched other than minimum documentation to the extent that such documents are included in the fields searched
Published examined utility model applications of Japan     1922–1996
Published unexamined utility model applications of Japan     1971–2021
Registered utility model specifications of Japan     1996–2021
Published registered utility model applications of Japan     1994–2021

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | WO 2018/155390 A1 (HISAMITSU PHARMACEUTICAL CO., INC.) 30 August 2018 (2018-08-30) comparative examples 2, 3, 6-11 | 1-7 |
| A | JP 2019-525932 A (CORIUM INTERNATIONAL, INC.) 12 September 2019 (2019-09-12) | 1-7 |
| A | JP 2008-266175 A (HISAMITSU PHARMACEUTICAL CO., INC.) 06 November 2008 (2008-11-06) | 1-7 |

☐ Further documents are listed in the continuation of Box C.     ☒ See patent family annex.

| | | |
| --- | --- | --- |
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| 05 January 2021 (05.01.2021) | 19 January 2021 (19.01.2021) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**
Information on patent family members

International application No.

PCT/JP2020/043382

| Patent Documents referred in the Report | Publication Date | Patent Family | Publication Date |
|---|---|---|---|
| WO 2018/155390 A1 | 30 Aug. 2018 | US 2020/0121611 A1 comparative examples 2, 3, 6-11 EP 3586841 A1 CN 110300582 A KR 10-2019-0112810 A TW 201834641 A | |
| JP 2019-525932 A | 12 Sep. 2019 | US 2018/0028663 A1 WO 2018/022816 A1 EP 3490540 A1 | |
| JP 2008-266175 A | 06 Nov. 2008 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010134433 A
- WO 2012165253 A
- WO 2018155390 A
- WO 2012165254 A

**Non-patent literature cited in the description**

- Japanese Pharmaceutical Excipients Directory. International Pharmaceutical Excipients Council Japan, 2016